# EUROPEAN PATENT APPLICATION

(11) **EP 4 579 610 A1**
(43) Date of publication of application: **02.07.2025**
(21) Application number: 24219444.7
(22) Date of filing: 12.12.2024
(51) Int. Cl.: G06V 10/62, A61B 5/11, G06V 10/764, G06V 10/80, G06V 20/40, G06V 20/52, G06V 40/20

(54) **AUTOMATED VIDEO TRACING OF PLAYERS USING INERTIAL SENSORS**

(30) Priority: 29.12.2023 US 202318401031
(71) Applicant: adidas AG, 91074 Herzogenaurach (DE)
(72) Inventor: DUEMLER, Burkhard, Herzogenaurach 91074 (DE)
(74) Representative: Bardehle Pagenberg Partnerschaft mbB Patentanwälte Rechtsanwälte

(57) **Abstract**

A method of tracking video objects within video footage includes receiving, at a computing device, video footage of a space and event data from a plurality of monitors. Each monitor includes a motion sensor configured to measure movements of a respective monitored video object within the space. The event data includes motion events transmitted by the monitors. The method also includes using a machine learning model to identify and track the monitored video objects within the video footage and to identify, from the video footage, video events performed by the monitored video objects. The method also includes using the computing device to assign a respective persistent identifier to each of the monitored video objects within the video footage based at least in part on commonality between video events performed by the monitored video object and motion events transmitted by one of the monitors.

## Description

### FIELD

The described embodiments generally relate to use of motion data acquired from by monitors on moving objects in conjunction with video footage of the moving objects to improve object tracking and motion analytics, especially in the context of tracking multiple overlapping objects (e.g., monitoring a team of players).

### BACKGROUND

It has become common practice in the field of professional athletics to record athletes' activity both within matches and during preparation for matches for the purpose of analyzing the athletes' performance and optimize training. Recorded information for this purpose can include video footage of an area in which athletes are active as well as measurements of particular quantitative factors of individual athletes. For example, athletes may wear motion sensors or biometric sensors while engaged in a match or when training to facilitate analysis of the athlete's individual performance.

Video footage can be enhanced by applying image analysis tools to find athletes within the video footage and apply trackers - known colloquially as "tracklets," - to those athletes. Existing image analysis tools can lose track of athletes in conditions that obscure the athletes from view, such as when athletes leave frame, collide with one another, when there are multiple moving and crisscrossing objects within the same frame, or move behind other objects within the field of view. Additionally, existing image analysis tools are typically not sufficient to reliably identify athletes by their distinguishing features such as jersey number or facial appearance, especially when there are multiple athletes being tracked and monitored. As a result, existing tools can struggle to track athletes persistently throughout a sporting event.

### BRIEF SUMMARY

Systems and methods herein apply machine learning technology to identify video events within video footage. Systems and methods herein further coordinate the identified video events with motion data acquired by wearable monitors worn by athletes within the video footage for analysis purposes. For example, some processes herein relate to assessing the similarity between video events in the video footage as identified by the machine learning model to the data transmitted by the wearable monitors. The results of such assessments may be used in some embodiments to determine which monitor 110 is worn by which identified athlete at a certain point or certain points within the duration of the video footage. The determination can be used, in some embodiments, to correct time information within the data transmitted by the wearable monitors, thereby overcoming potential inaccuracies in the monitors' timekeeping. The determination can further be used, in some embodiments, to facilitate tracking athletes persistently throughout the video footage, such as by confirming identity of athletes before and after events that obscure the athletes from view within the video footage.

Some aspects of the present disclosure are directed to a method of tracking objects within video footage. The method can comprise receiving, at a computing device, video footage of a space and event data from a monitor. The monitor can comprise a motion sensor configured to measure movements of a monitored video object within the space. The event data can comprise motion events transmitted by the monitor. The method can also comprise using a machine learning model on the computing device to identify, from the video footage, video events performed by the monitored video object. The method can also comprise assigning a respective persistent identifier to the monitored video object within the video footage based at least in part on commonality between video events performed by the monitored video object and motion events transmitted by the monitor.

In some embodiments according to the foregoing, the monitored video object can be one among multiple video objects. The monitor can be one among multiple monitors that each comprise a motion sensor configured to measure movements of a respective monitored video object. The method can comprise assigning a respective persistent identifier to each of the multiple monitored video objects. Assigning the respective persistent identifiers can comprise assigning respective preliminary identifiers to the monitored video objects within the video footage at multiple different times within the video footage. Assigning the respective persistent identifiers can also comprise finding preliminary identifiers assigned to a same monitored video object within different portions of the video footage based at least in part on commonality between video events performed by the same monitored video object and motion events transmitted by one of the monitors. Assigning the respective persistent identifiers can also comprise consolidating preliminary identifiers found to be assigned to the same monitored video object into a single preliminary identifier. Assigning the respective persistent identifiers can also comprise, after the consolidating step, converting the preliminary identifiers to the persistent identifiers.

In some embodiments according to any of the foregoing, assigning the respective preliminary identifiers can comprise assigning a new preliminary identifier to a particular one of the monitored video objects at a discontinuity point within the video footage at which the computing device cannot determine, based on the video footage, identity between the particular one of the monitored video objects and any of the monitored video objects to which a preliminary identifier was assigned within an earlier portion of the video footage.

In some embodiments according to any of the foregoing, assigning the respective preliminary identifiers can comprise identifying key moments within the video footage, wherein every monitored video object is identifiable by the machine learning model from the video footage alone at each key moment. Assigning the respective preliminary identifiers can also comprise calculating similarities between motion events among the event data transmitted by each wearable monitor and video events performed by each monitored video object to which a preliminary identifier is assigned during at least one key moment.

In some embodiments according to any of the foregoing, the method can comprise processing motion data captured by the motion sensor of the wearable monitor to identify the motion events before transmitting the event data to the computing device.

In some embodiments according to any of the foregoing, the machine learning model can be a first machine learning model. The processing of the motion data can comprise applying a second machine learning model hosted by the wearable monitor to the motion data.

In some embodiments according to any of the foregoing, the method can comprise identifying, for each persistent identifier, a corresponding one of the monitors that comprises the motion sensor configured to measure the movements of the monitored video object to which the persistent identifier is assigned.

In some embodiments according to any of the foregoing, the method can comprise applying a timestamp to one of the motion events transmitted by the corresponding one of the monitors matching a time when a corresponding video event is performed by the monitored video object to which the persistent identifier is assigned.

In some embodiments according to any of the foregoing, the method can comprise applying a timestamp to at least one of the motion events to match a time at which one of the video events occurred.

In some embodiments according to any of the foregoing, the event data can comprise preliminary timestamps applied to the motion events by the monitor, and applying the timestamp to at least one of the motion events comprises replacing one of the preliminary timestamps with a final timestamp.

In some embodiments according to any of the foregoing, the video objects can be athletes engaged in a sporting event.

In some embodiments according to any of the foregoing, each athlete can wear one of the monitors.

In some embodiments according to any of the foregoing, the motion events can comprise any one or any combination of kicking, running, walking, and standing.

In some embodiments according to any of the foregoing, the motion events can comprise magnitude information in the form of any one or any combination of kick force, distance traveled, and speed.

In some embodiments according to any of the foregoing, the monitor can comprise a controller configured to identify the motion events from the motion sensor of the monitor.

Some aspects of the present disclosure are directed to a system. The system can comprise a wearable monitor comprising a motion sensor. The system can also comprise a computing device configured to receive event data transmitted by the wearable monitor. The event data can comprise motion events. The computing device can comprise a non-transitory computer readable medium on which a machine learning model is stored. The machine learning model can be configured to identify video events from video footage. The computing device can be configured to correlate the video events to the motion events.

In some embodiments according to any of the foregoing, the wearable monitor can be configured to be integrated into an article of wear.

In some embodiments according to any of the foregoing, the article of wear can be a shoe insole.

In some embodiments according to any of the foregoing, the wearable monitor can comprise a motion sensor and a controller configured to identify the motion events from measurements acquired by the motion sensor.

In some embodiments according to any of the foregoing, the machine learning model can be configured to track video objects within video footage based on the event data.

In some embodiments according to any of the foregoing, the machine learning model can be a first machine learning model, and each wearable monitor among the plurality of wearable monitors hosts a second machine learning model configured to identify the motion events from motion data acquired by the motion sensor of the wearable monitor.

In some embodiments according to any of the foregoing, the wearable monitor can be a first wearable monitor, the system comprises a plurality of wearable monitors that includes the first wearable monitor, each wearable monitor among the plurality of wearable monitors comprises a motion sensor, and the computing device is configured to receive event data transmitted by the wearable monitors.

### BRIEF DESCRIPTION OF THE DRAWINGS/FIGURES

FIG. 1A shows a system according to some aspects of the present disclosure.
FIG. 1B shows a portion of video footage processed by the system of FIG. 1A.
FIG. 1C is a flowchart of a process carried out by a wearable monitor according to some aspects of the present disclosure.
FIG. 1D is a flowchart of a process carried out by a computing device according to some aspects of the present disclosure.
FIG. 2A is a graph of video analysis data according to some aspects of the present disclosure.
FIG. 2B is a graph of event data according to some aspects of the present disclosure.
FIG. 2C is a flowchart of a method of correcting time data according to some aspects of the present disclosure.
FIG. 3A is a flowchart of a process of analyzing video analysis data and event data according to some aspects of the present disclosure.
FIG. 3B is an illustration of a hypothesis tree according to some aspects of the present disclosure.
FIG. 4 is a flowchart of a matching analysis according to some aspects of the present disclosure.
FIG. 5 is a flowchart of a process of analyzing video analysis data according to some aspects of the present disclosure.

### DETAILED DESCRIPTION

The concepts of the present disclosure will be described in detail with reference to embodiments thereof as illustrated in the accompanying drawings. References to "some embodiments", "one embodiment", "an embodiment", "an exemplary embodiment", etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described.

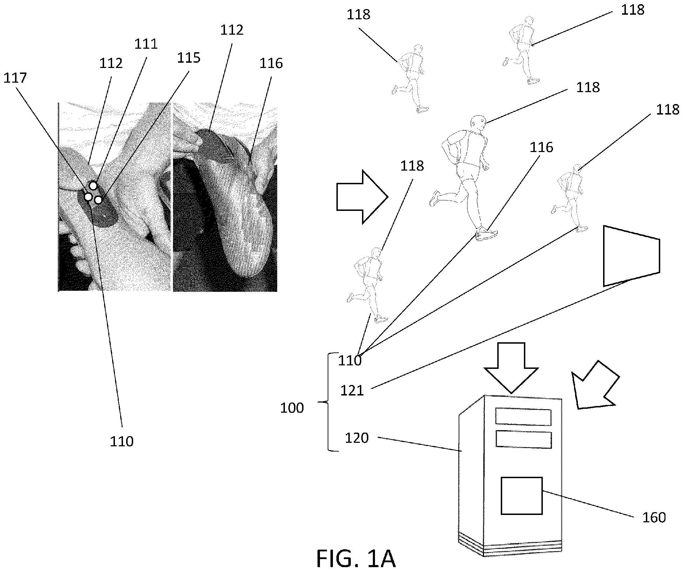

FIG. 1A illustrates a system 100 comprising one or more monitors 110 and a computing device 120. Each monitor 110 can be a wearable monitor. Monitor 110 can be configured to generate activity determinations of a wearer, such as athlete 118, of monitor 110 based on motion of the wearer. Computing device 120 stores a machine learning model 160 trained to interpret video footage.

Monitor 110 can be a wearable monitor. Monitor 110 can comprise a sensor 111 and a controller 115. Because monitor 110 can be a wearable monitor, sensor 111 can be a wearable sensor. Similarly, controller 115 can be a wearable controller.

Sensor 111 can comprise a motion sensor configured to measure athlete's 118 motions. The motion sensor can comprise, for example, any one or any combination of an accelerometer, a magnetometer, a gyroscope, a global positioning system ("GPS"), a pedometer, a posture sensor, an impact sensor, a pressure sensor, and a plurality of any of the foregoing.

Controller 115 can be configured identify motion events from motion data captured by sensor 111. Because monitor 110 comprises controller 115, monitor 110 according to some embodiments can be configured to identify motion events from measurements made by sensor 111, wherein the motion events comprise either or both of actions performed by athlete 118 among a plurality of predefined motion types and performance metrics of athlete 118 among a plurality of predefined metrics. In some embodiments, monitor 110 can further be configured to determine, or at least approximate, a time at which the identified motion event occurred. Thus, for example, in some embodiments wherein monitor 110 identifies a motion event that includes athlete 118 kicking a ball, monitor 110 can further be configured to determine, or at least approximate, when athlete 118 kicked the ball. In some embodiments, motion types can comprise, for example, any one or any combination of kicking, running, walking, and standing. In some embodiments, metrics can comprise magnitude information. In some embodiments, the magnitude information can comprise, for example, any one or any combination of kick force, distance traveled, and speed.

In some embodiments, monitor 110 may host a machine learning model 117 to process the motion data captured by sensor 111 and generate the motion determinations. Similarly to machine learning model 160 of computing device, the machine learning model 117 of monitor 110 could be any of a variety of types of machine learning model 117. In some embodiments, the machine learning model 117 of monitor 110 is a neural network. In some embodiments, motion data captured by monitor 110 can further include timestamps which identifies a time at which the captured motion was recorded. Thus, for example, in some embodiments wherein monitor 110 generates a motion determination that athlete 118 kicked a ball, monitor 110 can further be configured to determine when athlete 118 kicked the ball based on the corresponding timestamps.

In some embodiments, monitor 110 can be configured to be integrated into an article of wear. The article of wear can comprise, for example, a shoe 116, a wristband, glove, a shirt, a headband, a hat, or any other article of wear. Monitor 110 of the illustrated embodiment can be integrated into shoe 116 by inserting monitor 110 into an insole 112, then disposing insole 112 within shoe 116. Athlete 118 can therefore wear monitor 110 of the illustrated embodiment by wearing shoe 116 while monitor 110 remains inserted in insole 112 and insole 112 remains disposed within shoe 116. Monitor 110 according to other embodiments can be integrated into shoe 116 or other articles of wear by any processes and tools appropriate for the type of article and the type of activity for which monitor 110 is intended.

Computing device 120 can comprise a processor and a non-transitory computer readable medium, wherein the non-transitory computer readable medium carries instructions that, when read by the processor, cause the processor to interpret video footage. The instructions stored on the medium can comprise part of the machine learning model 160. Machine learning model 160 according to various embodiments can be any type of machine learning model 160. Thus, machine learning model 160 in some embodiments can be, for example, a neural network.

In some embodiments, computing device 120 can be remote from monitor 110. That is, computing device 120 can be a separate physical device from monitor 110. Accordingly, in some embodiments, computing device 120 can comprise, for example, comprises any one or any combination of a smart device, a laptop computer, a desktop computer, and a cloud computing system. In some embodiments, computing device 120 can be single device. In other embodiments, computing device 120 can be a distributed group of cooperating devices.

In some embodiments, when implemented with a controller 115, monitor 110 can further be configured to identify motion events based on the motion data captured over a predetermined time period. Non-limiting examples of motion data include but are not limited to speed, angular velocity, acceleration, and location. Non-limiting examples of motion events include but are not limited to kicking, traveling at a rate, and being at a location. Controller 115 can be configured to convert motion data into event data according to the type of object monitored and how the monitor is intended to be applied to the monitored object. For example, where monitor 110 is configured to monitor an athlete 118 while positioned in a shoe 116, controller 115 may be configured such that speed within motion data does not correspond exactly to a magnitude of a rate of travel motion event because a foot of an athlete 118 can move at a different speed than the athlete 118 overall. Controller 115 can collect identified motion events 155 together with timing of the motion events 155 into event data. Event data can therefore comprise a plurality of motion events identified from motion data as well as time information for the motion events. As explained further below, controller 115 according to some embodiments creates the time information in view of a local clock, such as a clock included in monitor 110 or a clock of a mobile device monitor 110 communicates with. In some instances, the local clock used for this purpose may not be perfectly accurate. As a result, the time information within event data according to some embodiments can comprise the true times at which motion events occurred, while event data according to further embodiments can comprise approximations of the times at which motion events occurred. The time information within event data according to further embodiments can comprise timing of motion events relative to one another without reference to clock time. Monitor 110 can further be configured to transmit event data comprising the motion events to computing device 120. In such embodiments, the machine learning model 160 stored on computing device 120 can then operate on hardware separate from monitor 110.

Monitor 110 according to various embodiments can be configured to communicate with computing device 120 to transmit event data through any electronic communication processes, hardware, and protocols. In further embodiments, monitor 110 can be configured to transmit event data to computing device 120 through a physical electronic connector. In further embodiments, monitor 110 can be configured to transmit event data to computing device 120 through one or more wires electronically connecting monitor 110 to device 120. Monitor 110 in some embodiments can be configured to transmit event data to computing device 120 as the event data are identified in real time, such as while monitor 110 is being worn by an active athlete 118. Monitor 110 in further embodiments can be configured to transmit event data to computing device 120 in a batch, such as after athlete has completed participation in one or more activities while wearing monitor 110. In further embodiments, athlete monitor 110 can be configured to transmit information to an intermediary, and the intermediary can be configured to transmit information to computing device 120. The intermediary can be, for example, a smart phone, a cloud computing system, or any other computing device. In some embodiments, athlete monitor 110 can transmit the motion determinations to the intermediary, and the intermediary can then transmit the motion determinations to computing device. In further embodiments, athlete monitor 110 can be configured to transmit the motion data to the intermediary, the intermediary can host a machine learning model configured to derive the motion determinations from the motion data, and the intermediary can be configured to transmit the motion determinations derived by the machine learning model to computing device 120. Thus, all processes described herein as being carried out by processor 115 could, in other embodiments, be carried out on an intermediary device instead.

System 100 according to some embodiments can further comprise one or more cameras 121. Camera 121 can be configured to capture video data of a space over a predetermined time period. Video data can comprise video footage, such as video footage 122 discussed further below. In some embodiments, video data can also comprise time information, such as the time at which the video footage was captured. In some embodiments, the predetermined time period of captured video footage corresponds in time with the predetermined time period of the captured motion data from monitor 110. In this manner, video events can be correlated to motion events to increase the accuracy of video object detection within the video data. Video events include identified actions within the video data and associated with a video object within the video data. Examples of video events include but are not limited to an action event (e.g., a kick, kick force, a pass), distance traveled (e.g., over a predetermined period of time), a movement path (e.g., over a predetermined period of time), and movement speed (e.g., over a predetermined period of time within the video data). Because camera 121 can capture video data of a space wherein a monitor 110 is used to monitor a video object, such as an athlete 118, during the same predetermined time that monitor 110 is used to monitor the video object, events performed by the video object or happening to the video object may be detectable within both the video data captured by camera 121 and the motion data captured by sensor 111 of monitor 110. Thus, by processing the motion data to identify motion events as discussed above with regard to monitor 110 and by processing the video data to identify video events, it may be possible to synchronize the motion data with the video data and to determine which video object detectable in the video data the monitor 110 was applied to. Camera 121 according to some embodiments can further be configured to transmit the video footage to computing device 120.

System 100 according to some embodiments can be configured to track video objects within video footage. Video objects can be athletes 118 within some embodiments described herein. Thus, any process described herein with respect to athletes 118 is an example of a concept that can be applied to tracking video objects in video generally. Moreover, processes described herein with respect to tracking video objects in video can be used to track athletes in video.

Computing device 120 according to some embodiments can further be configured to identify, from video footage, video events performed by video objects within the video footage. Within system 100, each monitor 110 can be configured to track motion data and measure movements of its corresponding video object based on the tracked motion data while camera 121 captures video footage of the video objects. In other words, system 100 may include synchronous motion detection and video capture functionality and system 100 may be configured to correlate motion data from the motion detection with video data provided from the video capture functionality to increase the accuracy of object detection within video data.

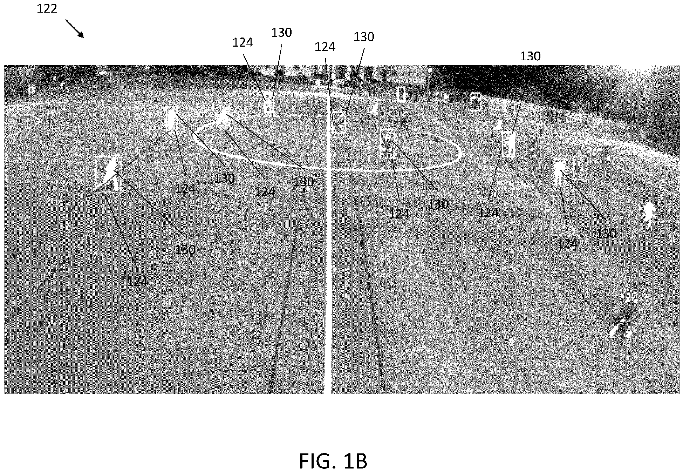

FIG. 1B shows an exemplary frame of video footage 122 with identifiers 124 applied thereto. Video footage 122 is comprised by the video data captured by camera 121. Multiple video objects 130 (athletes 118 in the illustrated example) are detectable within video footage 122. Machine learning model 160 according to some embodiments can assign each detectable video object 130 an identifier 124 and annotate video footage 122 with the resulting identifier 124 as shown. Identifiers 124 can highlight video objects 130 and associated motion data (e.g., actions, movement speed, movement path) within video footage 122, thereby making video footage 122 easier to interpret. Identifiers 124 can also facilitate analysis of individual video objects 130, such as by enabling computer analytics to be tied to individual identifiers 124. Identifiers 124 can each have a unique tag, such as a serial number, differentiating them from one another. Identifiers 124 can also be given distinguishing visual characteristics to further contribute to ease of review of video footage 122. Thus, in some embodiments, identifiers 124 can each be given a different color, which make different video objects 130 easily distinguishable to the human eye. In further embodiments, identifier 124 can be given different colors depending on detectable characteristics of the video objects 130. For example, in some embodiments wherein the video objects 130 are athletes 118 in a team sport, computing device 120 may be able to detect a jersey color of video objects 130 and assign identifiers 124 with colors that differ according to jersey color.

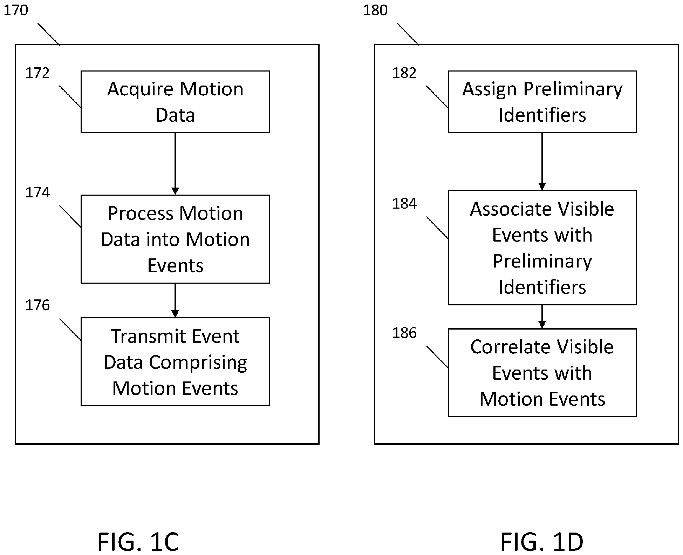

Turning to FIGS. 1C and 1D, monitor 110 and computing device 120 can conduct different analytical processes 170, 180 to correlate motion events identifiable from motion data with video events identifiable from video footage 122. Referring specifically to FIG. 1C, monitor 110 can be configured to perform analytical process 170. Analytical process 170 can comprise an acquiring step 172. Acquiring step 172 can comprise using sensor 111 to acquire motion data of the video object 130, such as athlete 118, wearing the monitor 110 at an acquiring step 172.

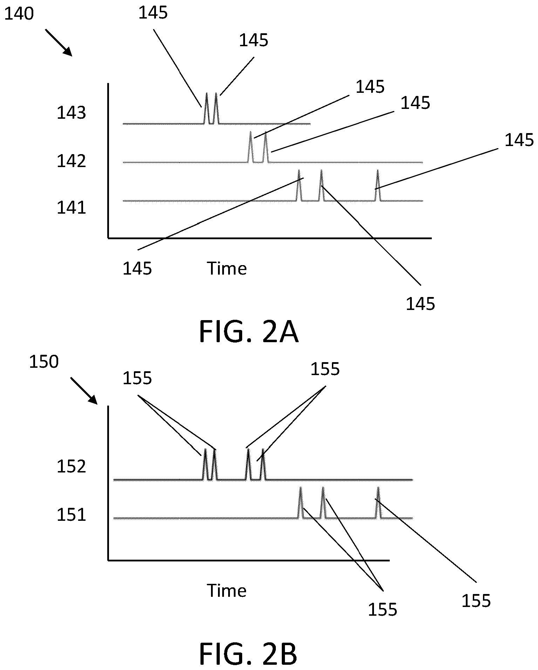

At a processing step 174, the controller 115 of a monitor 110 can process the motion data captured by the sensor 111 of the same monitor 110 to create event data. In some embodiments, the event data created in processing step 174 can be the event data 150 described below with regard to FIG. 2B. Accordingly, processing the motion data within processing step 174 can comprise identifying motion events from the motion data, such as the motion events 155 described below. As noted above, in some embodiments, each controller 115 can host a machine learning model 117 that is distinct from machine learning model 160 of computing device 120. The machine learning model 117 hosted by controller 115 can be trained to identify motion events 155 from motion data. Accordingly, in some embodiments, processing the motion data within processing step 174 can comprise identifying motion events 155 from motion data by applying the machine learning model 117 hosted by controller 115 to the motion data.

At a reporting step 176, monitor 110 can transmit the event data 150 created in processing step 174 to computing device 120.

Turning to FIG. 1D, computing device 120 can be configured to perform a process 180. Process 180 can comprise an assignment step 182. Assignment step 182 can comprise using computing device 120 to assign identifiers 124 to video objects 130 detectable in video footage 122 as described above. In some embodiments, computing device 120 can use machine learning model 160 to assign identifiers 124. Thus, machine learning model 160 according to some embodiments can be trained to identify video objects 130 within video footage 122. In some embodiments, identifiers 124 applied in assignment step 182 can be preliminary identifiers to be converted to persistent identifiers in a later process as described below.

An associating step 184 can comprise using computing device 120 to associate video events with identifiers 124. As noted above, machine learning model 160 can be trained to identify video events, such as video events 145 described below with regard to FIG. 2A, from video footage 122. Accordingly, associating step 184 according to some embodiments can comprise further using machine learning model 160 to associate the identified video events 145 with the identifiers 124 assigned to the same video object 130 that performed the video events 145. Video events 145 can be identified prior to associating step 184 or during associating step 184. In some embodiments, video data 140 as described further below can comprise both video events 145 and the associations between video events 145 and identifiers 124.

FIGS. 2A and 2B are exemplary event that can be compared to correlate identifiers 124 to monitors 110. FIG. 2A shows an example of video analysis data 140. Video analysis data 140 comprises information created by analyzing video data captured by camera 121. Thus, while the video data captured by camera 121 can comprise video footage 122 itself, video analysis data 140 generated by computing device 120 can comprise identifiers 124, the positions of identifiers 124, video events 145, and associations between video events 145 and identifiers 124. In some embodiments, video analysis data 140 can further comprise one or more portions of the video data itself. Machine learning model 160 can be trained to create video analysis data 140 from video footage 122. Video analysis data 140 can comprise video events 145 detected by machine learning model 160 as being performed by video objects 130 within video footage 122. Video events 145 can be events among the same predefined types as the motion events identifiable by monitors 110. Video analysis data 140 created by machine learning model 160 can therefore comprise identifications of the same events transmitted by monitors 110 worn by video objects 130 that appear in video footage 122.

In some embodiments, machine learning model 160 can also be trained to associate video events 145 with the video objects 130 that are identified as performing the video events 145. In some embodiments, machine learning model 160 can associate video events 145 with the specific video objects 130 that performed the video events 145 by allocating each video event 145 to a one among a plurality of identifiers 141, 142, 143 (each alike to identifiers 124 described above) assigned to the video object 130 that performed the video event 145. Thus, as shown in FIG. 2A, machine learning model 160 may find that a video object 130 to which third identifier 143 is assigned performs two video events 145, followed by a video object 130 to which a second identifier 142 is assigned performing two video events 145, followed by a video object 130 to which a first identifier 141 is assigned performing three video events 145.

In some embodiments, machine learning model 160 can be configured to retire identifiers when video objects 130 become obscured. In some embodiments, machine learning model 160 can be configured to retire an identifier every time machine learning model 160 cannot find the video object 130 to which the identifier was assigned within video footage 122. Retiring an identifier as soon as machine learning model 160 cannot find the video object 130 to which the identifier was assigned can be considered a "fast" retirement. In other embodiments, machine learning model 160 can be configured to retire an identifier only if the video object 130 to which the identifier was assigned cannot be located with a threshold amount of certainty within an amount of time after the video object 130 becomes undetectable within video footage 122. Retirement of an identifier only if the video object 130 to which the identifier was assigned cannot be located with the threshold amount of certainty within an amount of time after the video object 130 becomes undetectable can be considered a "slow" retirement. Machine learning model 160 according to some embodiments can be configured or instructed to enact either fast or slow retirement. Machine learning model 160 can further be configured to create a new identifier whenever machine learning model 160 discovers a video object 130 without an identifier within video footage 122. As a result, different identifiers 141, 142, 143 may be associated with a same video object 130 at different times within video footage 122. Machine learning model 160 according to some embodiments can be trained to conduct further analysis to find pluralities of identifiers assigned at different times to a same video object 130. Thus, identifiers 141, 142, 143 at the stage of analysis represented in FIG. 2A can be considered preliminary identifiers.

Turning to FIG. 2B, event data 150 transmitted by monitors 110 to computing device 120 can comprise motion events 155 as well as the identity of the monitor 110 that transmitted each portion of event data 150. In the illustrated example, event data 150 transmitted by a first monitor 151 of the monitors 110 comprises three motion events 155. Also in the illustrated example, event data 150 transmitted by a second monitor 152 of the monitors 110 comprises four motion events 155.

Computing device 120 according to some embodiments can be configured to find pairs of preliminary identifiers assigned to same monitored video object 130 within different portions of video footage 122 based at least in part on commonality between video events 145 performed by the same video object 130 and motion events 155 transmitted by one of monitors 110, including monitors 151, 152. That is, computing device 120 can be configured to compare video analysis data 140 with event data 150 to find sequences of video events 145 having the most in common with sequences of motion events 155 transmitted by specific monitors 110, including monitors 151, 152. In some embodiments, assessing commonality can comprise, for example, matrix step 412 of matching analysis 400 as described further below. In further embodiments, assessing commonality can comprise analysis phase 408 of matching analysis 400 as described further below. In further embodiments, assessing commonality can comprises matching analysis 400 as described further below. Thus, in the illustrated example, computing device 120 may conclude from the similarity of the timing and pattern of video events 145 allocated to second identifier 142 and third identifier 143 to the timing and pattern of motion events 155 transmitted by second monitor 152 that both second identifier 142 and third identifier 143 were assigned at different times to a single video object 130 that wore second monitor 152. Computing device 120 may further conclude from the similarity of the timing and pattern of video events 145 allocated to first identifier 141 to the timing and pattern of motion events 155 transmitted by first monitor 151 that first identifier 141 was assigned to a video object 130 that wore first monitor 151. In some embodiments, computing device 120 can be configured to find pairs of preliminary identifiers assigned to same monitored video object 130 within different portions of video footage 122 by application of machine learning model 160. According to some such embodiments, machine learning model 160 can be trained to assess commonality between video analysis data 140 and event data 150. Accordingly, in some embodiments, any of the foregoing steps described as being performed by machine learning model with respect to making conclusions based on comparison of video analysis data 140 and event data 150 can be achieved by application of machine learning model 160.

After determining which preliminary identifier, or which plurality of identifiers 141, 142, 143 were assigned to a same video object 130 throughout video footage 122, machine learning model 160 can replace preliminary identifiers 141, 142, 143 with persistent identifiers assigned one-to-one to respective video objects 130. Machine learning model 160 can assign a persistent identifier to each monitored video object 130 within video footage 122 based at least in part on commonality between video events 145 performed by the monitored video object 130 and motion events 155 transmitted by one of the monitors 110, 151, 152. Thus, by using motion events 155 to consolidate multiple preliminary identifiers into a single persistent identifier, machine learning model 160 can track video objects 130 within video footage 122 based on event data 150. Replacing preliminary identifiers 141, 142, 143 with persistent identifiers can comprise consolidating any two or more preliminary identifiers 141, 142, 143 found to have been assigned to a same video object 130 at different times within video footage 122 into a single persistent identifier. Thus, in the illustrated example, machine learning model 160 may replace first preliminary identifier 141 with a persistent identifier that corresponds to first monitor 151. Further within the illustrated example, machine learning model 160 may consolidate second preliminary identifier 142 and third preliminary identifier 143 into a single persistent identifier that corresponds to second monitor 152.

Using computing device 120 to correlate video events 145 to motion events 155 by finding which identifiers, such as any the identifiers 124, 141, 142, 143 discussed above, correspond to which monitors 110, 151, 152 can also assist with correcting or assigning time information to event data 150. For example, in some embodiments, monitors 110 may transmit event data 150 with slightly inaccurate timestamps or with no timestamps at all. In some examples, monitors 110 according to some embodiments may have internal clocks that may not be perfectly synchronized to true time, resulting in slightly inaccurate timestamps. In further examples, monitors 110 may transmit event data indicating only how far apart the motion events detected by monitors 110 occurred, but without timestamps. Thus, upon matching a monitor 110, 151, 152 to at least one preliminary identifier 141, 142, 143, computing device 120 can apply a time offset to event data 150 received from that monitor 110. The time offset can be a constant amount of time added to or subtracted from the time within the event data 150 to synchronize the event data 150 with video analysis data 140. Each monitor 110, 151, 152 can have an independent clock, and may therefore be assigned a time offset of a different magnitude. The time offsets can then be used in matching steps 316, 516 and matching analysis 400 described below.

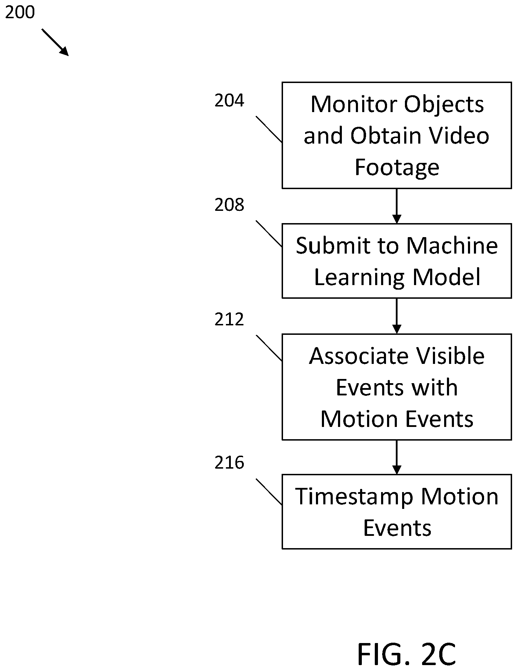

FIG. 2C shows a synchronizing method 200. A data acquisition step 204 comprises obtaining video footage 122 of video objects 130 wearing monitors 110, 151, 152. A submitting step 208 comprises submitting video footage 122 and event data 150 to machine learning model 160. An associating step 212 comprises determining which identifiers 141, 142, 143 correspond to which monitors 110, 151, 152 as described above with regard to FIG. 2B by using machine learning model 160 after submitting event data 150 and video footage 122 to machine learning model 160. A timestamping step 216 comprises, after machine learning model 160 determines which monitor 110, 151, 152 corresponds to which identifier or identifiers, machine learning model 160, using machine learning model 160 to apply a timestamp to video events 145 within event data 150 to match times when corresponding video events 145 appeared in video footage 122.

Video footage 122 may be accompanied by true time information, so timestamps applied based on when video events 145 appear in video footage 122 may, in some embodiments, match the true times at which those events occurred. The timestamps applied to motion events 155 transmitted by a specific one of the monitors 110, 151, 152 this way may therefore match the actual times when video events 145 were performed by the individual video object 130 to which the persistent identifier corresponding to that one of the monitors 110, 151, 152 was assigned. In embodiments wherein monitors 110, 151, 152 transmit event data 150 with inaccurate timestamps, machine learning model's 160 application of timestamps to event data 150 in view of video analysis data 140 can comprise correcting the inaccurate timestamps. In some embodiments, timestamps within event data 150 as transmitted by monitors 110, 151, 152 can be considered preliminary timestamps, and correcting inaccurate timestamps can comprise replacing the preliminary timestamps associated with particular motion events 155 with final timestamps that match the time of occurrence of corresponding video events 145.

Accordingly, in the illustrated example, machine learning model 160 can apply timestamps to motion events 155 transmitted by first monitor 151 matching the times at which the video object 130 to which first preliminary identifier 141 was assigned can be seen to perform corresponding video events 145 within video footage 122. Further according to the illustrated example, machine learning model 160 can apply timestamps to motion events 155 transmitted by second monitor 152 matching the times at which the video object 130 to which second identifier 142 and third identifier 143 were assigned can be seen to perform corresponding video events 145 within video footage 122.

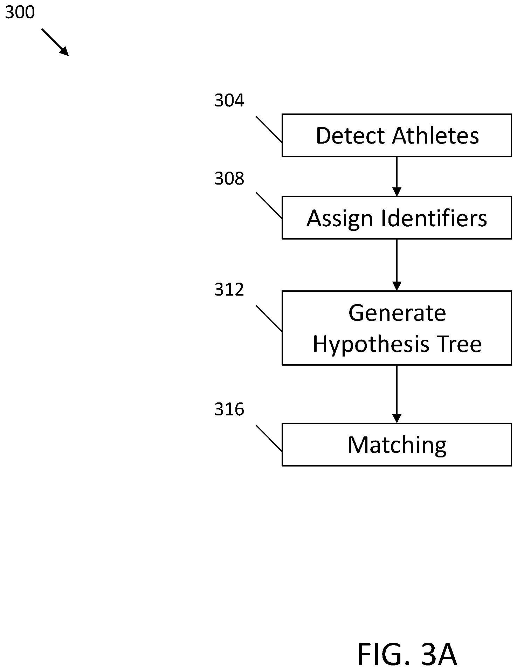

FIG. 3A illustrates a process 300 for consolidating preliminary identifiers into persistent identifiers. Within process 300, a detecting step 304 comprises using machine learning model 160 to detect video objects 130 within video footage 122. An assigning step 308 comprises assigning preliminary identifiers to the detected video objects 130. In some embodiments, machine learning model 160 can further be configured to cause preliminary identifiers to follow the video objects 130 to which they are assigned.

A discontinuity point may occur when a video object 130 whose previous identifier was retired becomes detectable within video footage 122. Such instances may be discontinuity points because machine learning model 160 may be unable to confirm, based on video footage 122 alone, identity between the re-detected video object 130 and any video object 130 to which a preliminary identifier was previously assigned. Thus, it may not be assumed that machine learning model 160 could reliably apply a same preliminary identifier both before and after such discontinuity points. Machine learning model 160 in some embodiments may therefore be configured to retire a preliminary identifier at any point within video footage 122 wherein machine learning model 160 cannot locate, based on video footage 122 alone, the video object 130 to which the preliminary identifier was assigned. Machine learning model 160 according to such embodiments may further be configured to generate a new preliminary identifier and assign the newly generated preliminary identifier to a detected video object 130 lacking a preliminary identifier at each discontinuity point. As a result, machine learning models 160 according to some embodiments may repeatedly retire preliminary identifiers and generate and assign new preliminary identifiers throughout the course of video footage 122 as video objects 130 move into and out of conditions obscuring them from video footage 122. An individual video object 130 may therefore be assigned a series of different preliminary identifiers over the course of video footage 122. Accordingly, assigning step 308 can comprise assigning respective preliminary identifiers to the monitored video objects within the video footage at multiple different times within the video footage

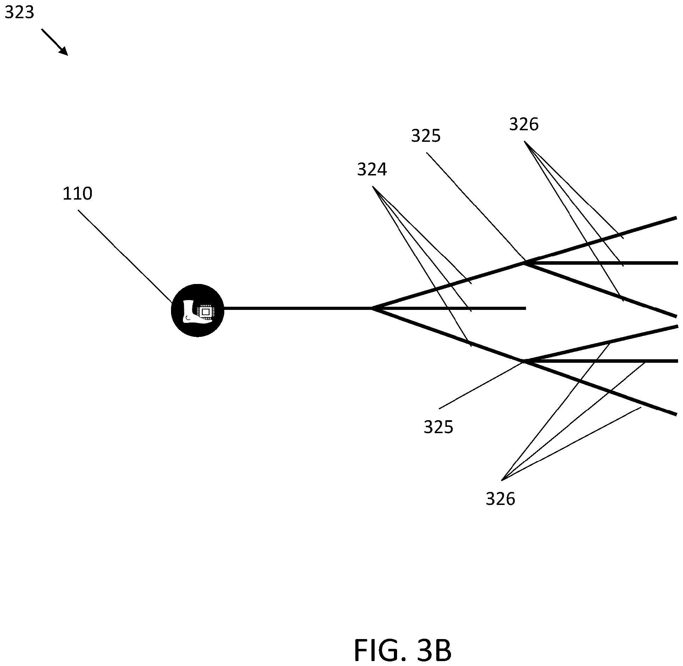

Generating step 312 can comprise generating a hypothesis tree for each monitor 110, such as hypothesis tree 323 shown in FIG. 3B. Each branch of hypothesis tree 323 represents a different preliminary identifier that could be assigned to a same video object 130 as a previous preliminary identifier based on machine learning model's 160 analysis of video footage 122. Thus, hypothesis tree 323 can comprise branches for first preliminary identifiers 324. Each first preliminary identifier 324 could possibly be the earliest preliminary identifier to be assigned to the video object 130 that wore the monitor 110 that provides the root of hypothesis tree 323.

Each branch in hypothesis tree ends at a retirement 325 of the preliminary identifier to which the branch corresponds. Retirement 325 may therefore correspond to a discontinuity point, meaning retirement 325 may be followed by multiple preliminary identifiers that could possibly have been assigned to a same video object 130 as the retired preliminary identifier. Thus, as shown in the example of FIG. 3B, each first preliminary identifier 324 may end at a retirement 325. Further, a retirement 325 of a first preliminary identifier 324 may be followed by multiple second preliminary identifiers 326 that could possibly have been assigned to a same video object 130 as the retired first preliminary identifier 324. Each second preliminary identifier 326 may eventually meet its own retirement 325, which may be followed by one or more third preliminary identifiers that could possibly have been assigned to a same video object 130 as the retired second preliminary identifier 326, and so on until an end of video footage 122. Any number of retirements 325 and new preliminary identifiers may be generated according to the events within video footage 122. Generally, any preliminary identifier generated and assigned after retirement of an earlier preliminary identifier could possibly be assigned to a same video object 130 as the retired preliminary identifier.

Returning to FIG. 3A, a matching step 316 can follow generating step 312. Matching step 316 can comprise analyzing video footage 122 together with event data 150 and hypothesis trees 323 to determine which preliminary identifier or preliminary identifiers were assigned to a same video object 130 as each monitor 110.

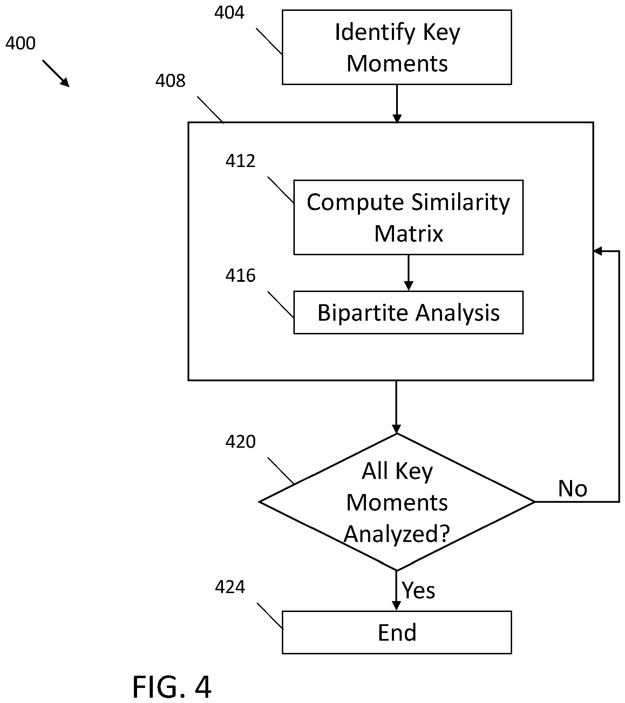

Matching step 316 can comprise matching analysis 400 as shown in FIG. 4. Matching analysis 400 comprises a key moment identification step 404. Key moment identification step 404 can comprise identifying key moments within video footage 122. Key moments in this context are moments within video footage 122 at which every monitored video object 130 is detectable within frame by machine learning model 160. Thus, key moments according to some embodiments can comprise portions within video footage 122 during which a number of video objects 130 detectable within frame is at least as great as a number of monitors 110 active during the event recorded by video footage 122. Key moments are separated by portions of video footage 122 during which less than all monitored video objects 130 are identifiable as such by machine learning model 160. At least one preliminary identifier may be retired at the end of every key moment. Additionally, each key moment begins with detecting at least one previously undetected video object 130, meaning key moments can co-occur with discontinuity points as described above.

Each key moment identified in key moment identification step 404 is separately analyzed in an analysis phase 408. Analysis phase 408 for each key moment comprises a matrix step 412. Matrix step 412 can comprise computing a matrix of similarities between video events 145 associated with each preliminary identifier active at that key moment and motion events 155 associated with each monitor 110. Thus, an element of the matrix may exist for each pair of one monitor 110 and one of the preliminary identifiers active at the relevant key moment. Similarity may be computed in any of a variety of ways. In some examples, similarity may be computed as the mean squared error between the motion events 155 of the monitor 110 of the matrix element and the video events 145 of the preliminary identifier of the same matrix element. In some embodiments, pairs of one monitor 110 and one of the preliminary identifiers that do not correspond to any branch of any hypothesis tree 323, or that conflict with the results of any already-completed bipartite analysis 416 in view of analysis trees 323, can be omitted as elements of the matrix.

Analysis phase 408 can also comprise a bipartite analysis step 416 after the matrix step 412. Bipartite analysis step 416 can comprise applying a bipartite matching process to the matrix created in matrix step 412. The bipartite analysis can be conducted in any of a variety of ways. In some examples, the bipartite analysis can be conducted according to a linear assignment method. Reference can be made to hypothesis trees 323 before conducting the bipartite matching process. Accordingly, any matrix elements corresponding to a pair of one monitor 110 and one preliminary identifier that is not possible within any hypothesis tree can be disregarded for the purpose of the bipartite analysis. Thus, pairs of preliminary identifiers and monitors 110 that are not possible can be excluded from consideration in the bipartite matching process.

Analysis phase 408 is conducted separately for each key moment identified in key moment identification step 404. Accordingly, for each analysis phase 408 conducted after the first analysis phase 408 for a single video footage 122, previously conducted analysis phases 408 can be taken into account in bipartite analysis step 416. For example, each completed analysis phase 408 identifies some branches in some hypothesis trees 323 as true. Thus, for subsequent analysis phases 408, previous analysis phases 408 can be assumed correct, thereby foreclosing other branches of the hypothesis trees 323. Bipartite analysis step 416 can therefore comprise excluding from consideration any pairs of one preliminary identifier and one monitor 110 that is not compatible with all previously completed analysis phases 408 in view of hypothesis trees 323.

Upon completing analysis phase 408, a check 420 may be conducted for whether an analysis phase 408 has been completed for each key moment. If an analysis phase 408 has not been completed for each key moment, matching analysis 400 can proceed to an analysis phase 408 for a next of the key moments. In some embodiments, analysis phases 408 are performed in chronological order of the key moments. Hypothesis trees 323 will tend to have fewer branches at earlier key moments than at later key moments. Thus, by performing analysis phases 408 in chronological order of the key moments, computational resources can be conserved by using conclusions from each analysis phase 408 to eliminate branches from consideration in later analysis phases 408.

If it is determined at check 420 that an analysis phase 408 has been completed for each key moment, matching analysis 400 can proceed to an end step 424. End step 424 can comprise consolidating all preliminary identifiers found to correspond to a same monitor 110 into one persistent identifier. Thus, performing analysis phase 408 for each key moment can find preliminary identifiers assigned to a same monitored video object 130, and end step 424 can comprise converting the preliminary identifiers found to be assigned to the same monitored video object 130 into a single persistent identifier. Each resulting persistent identifier can be assigned to a single video object 130 throughout video footage 122. Machine learning model 160 may annotate video footage 122 with the persistent identifiers to cause each persistent identifier to follow the video object 130 that wears the corresponding monitor 110 throughout video footage 122.

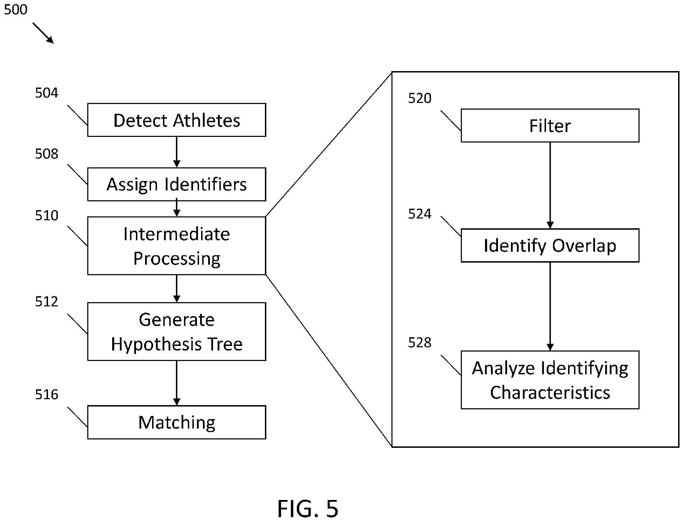

FIG. 5 shows a process 500 according to further embodiments. Process 500 according to some embodiments can be generally alike to process 300 described above. Thus, process 500 comprises a detecting step 504, an assigning step 508, a generating step 512, and a matching step 516 that may be alike in some or all respects to detecting step 304, assigning step 308, generating step 312, and matching step 516 as described above. Thus, like matching step 316 described above, matching step 516 can comprise matching analysis 400.

Process 500 further comprises an intermediate processing step 510 between assigning step 508 and generating step 512. Intermediate processing step 510 comprises using computing device 120 to analyze to video footage 122 to consolidate some pluralities of preliminary identifiers into single preliminary identifiers before generating the hypothesis tree. Accordingly, generating step 512 can comprise generating the hypothesis tree from only the preliminary identifiers that remain after intermediate processing step 510. In some embodiments, intermediate processing step 510 can be conducted without use of event data 150. In further embodiments, intermediate processing step 510 can be conducted relying on video footage 122 and an expected number of monitors 110 as the sole inputs. In some embodiments, computing device 120 can perform intermediate processing step 510 by applying machine learning model 160. In further embodiments, computing device 120 can perform intermediate processing step 510 without use of machine learning model 160.

In process 500, assigning step 508 can comprise retiring a preliminary identifier at any point where the video object 130 to which the preliminary identifier was assigned becomes undetectable within video footage 122, then assigning a new preliminary identifier upon detecting any video object 130 that was not detectable in the immediately preceding frame of video footage 122. Thus, in some embodiments, assigning step 508 can comprise assigning new preliminary identifiers upon detecting previously undetected video objects 130 without attempting to determine whether any of the previously undetected video objects 130 had, within an earlier portion of the video footage 122, been detected and assigned a subsequently retired preliminary identifier. Intermediate processing step 510 can comprise analyzing video footage 122 and the preliminary identifiers after assigning step 508 to find preliminary identifiers likely to have been assigned to a same video object 130 at different times and consolidating those preliminary identifiers into a single preliminary identifier for subsequent use in the generating step 512 and matching step 516.

Intermediate processing step 510 according to various embodiments can comprise any among a variety of analytical techniques and combinations thereof. Intermediate processing step 510 of the illustrated embodiment can comprise a filtering step 520 that comprises applying a filter to video footage 122, video analysis data 140, or both video footage 122 and video analysis data 140 to distinguish signal from noise. In some embodiments, the filtering step can be performed by computing device 120. The filter can be any type of filter capable of reducing noise, amplifying signals, or both, in video information. In some embodiments, the filter can be a Kalman filter. For example, a Kalman filter can be applied to groups of multiple frames or portions of video footage 122 to improve the accuracy of determinations of velocities of video objects 130. The improved accuracy determinations and any other information gained through filtering step 520 can be used in overlap step 524 and characteristics step 528 to consolidate preliminary identifiers. In some embodiments, the Kalman filter can be used to predict the position of an object by knowing its current speed and position. For example, video footage 122 may include data indicating that a first object has a velocity of zero and another object has a higher velocity (e.g., 12 km/h) passing the first object (overlapping); that video data may then be utilized to predict in the next frames where the moving object will continue. In this embodiment, the tracklet identifier of the first object can stay the same and the tracklet identifier of the moving object can also stay the same.

The results of applying the filter in filtering step 520 can be used to extend the preliminary identifiers. After extending the preliminary identifiers in view of the results of applying the filter, some preliminary identifiers may overlap by being assigned to a same video object 130 within a same portion of video footage 122. Overlap step 524 can comprise finding overlapping preliminary identifiers and consolidating them into a single preliminary identifier.

A characteristics step 528 can comprise reviewing video footage 122 for identifying characteristics exhibited by video objects 130, then consolidating any pluralities of preliminary identifiers assigned to athletes exhibiting mutually consistent identifying characteristics. Identifying characteristics in some embodiments can comprise characteristics such as jersey number and physical characteristics, such as facial features. In some such embodiments, even where video footage 122 does not enable computing device 120 to recognize jersey numbers or identifying physical characteristics consistently throughout video footage 122, computing device 120 may be able to identify specific instances at which a jersey number or identical physical characteristic becomes sufficiently clear to enable accurate identification by computing device. Accordingly, in some embodiments, computing device 120 can be configured to read jersey numbers at any points within video footage 122 wherein jersey numbers become clearly detectable, and characteristics step 528 can comprise using computing device 120 to consolidate any plurality of preliminary identifiers found to have been assigned to a video object 130 having the same jersey number within different portions of video footage 122. Similarly, in some embodiments, computing device 120 can be configured to recognize facial features of video objects 130 at any points within video footage 122 wherein facial features become clearly detectable, and characteristics step 528 can comprise using computing device 120 to consolidate any plurality of preliminary identifiers found to have been assigned to a video object 130 having the same facial features within different portions of video footage 122.

In some embodiments, identifying characteristics usable within characteristics step 528 can further comprise characteristics that may not be unique to a given video object 130 or consistent throughout video footage 122, but may be evaluated in conjunction with other circumstances to assess a likelihood that two different preliminary identifiers were assigned to a same video object 130 within different portions of video footage 122. Characteristics step 528 can therefore comprise calculating a similarity factor from the identifying characteristics and relevant circumstances by applying predetermined weights to the identifying characteristics and relevant circumstances, then consolidating two preliminary identifiers into a single preliminary identifier if the similarity factor exceeds a predetermined threshold. In some embodiments, identifying characteristics can comprise jersey color, location, velocity, or any combination of the foregoing. In some embodiments, relevant circumstances can comprise a numbers of video objects 130 wearing a same jersey color expected to be on field at once, a ratio of active preliminary identifiers within a portion of video footage 122 to an expected maximum number of preliminary identifiers, and recency of retirement of one or more preliminary identifiers. Thus, for example, within characteristics step 528, computing device 120 may determine that a later preliminary identifier appearing shortly after the retirement of an earlier preliminary identifier assigned to a video object 130 wearing the same jersey color in a nearby location is assigned to the same video object 130 as the earlier preliminary identifier if no other preliminary identifiers assigned to video objects 130 wearing the same jersey color were retired at nearly the same location and time. In further examples, where two preliminary identifiers assigned to two video objects 130 wearing different jersey colors are retired nearby to one another, then two new preliminary identifiers are assigned to the same two video objects 130 shortly thereafter, computing device 120 may be able to rely on the jersey colors to determine which new preliminary identifier was assigned to the same video object 130 as either of the retired preliminary identifiers. In further examples, within characteristics step 528, if an preliminary identifier is retired while assigned to a video object 130 traveling at a velocity and a later preliminary identifier appears shortly after the retirement at a location near where the video object 130 would arrive if the video object 130 continued to travel at the velocity for a duration of a gap between the retirement of the earlier preliminary identifier and the appearance of the later preliminary identifier, computing device 120 may determine that the earlier preliminary identifier and the later preliminary identifier were assigned to the same video object 130 if no other preliminary identifiers appeared or became retired at nearly the same location and time. Similarly, if a preliminary identifier is retired while assigned to a video object 130 that is stationary or traveling slowly, computing device 120 may determine that a preliminary identifier newly assigned to a video object 130 shortly after the retirement, but at a relatively large distance away from the last position of the retired preliminary identifier, is unlikely to have been assigned to the same video object 130 as the retired preliminary identifier.

In the illustrated embodiment, characteristics step 528 occurs after filtering step 520 and overlap step 524. However, in other embodiments, characteristics step 528 can occur before, between, or during filtering step 520 and overlap step 524.

By applying the above described filtering step 520, overlap step 524, and characteristics step 528, intermediate processing step 510 according to some embodiments can reduce an active number of preliminary identifiers before process 500 arrives at generating step 512. Thus, in some embodiments, intermediate step 510 can make generating step 512 and matching step 516 less computationally intensive and more efficient. Intermediate processing step 510 according to other embodiments can comprise more or fewer similarly purposed step to the filtering step 520, overlap step 524, and characteristics step 528 of the illustrated embodiment.

Embodiments described herein also may be directed to computer program products comprising software stored on any computer useable medium. Such software, when executed in one or more data processing device, causes a data processing device(s) to operate as described herein. Embodiments described herein may employ any computer useable or readable medium. Examples of computer useable mediums include, but are not limited to, primary storage devices (for example, any type of random access memory), secondary storage devices (for example, hard drives, floppy disks, CD ROMS, ZIP disks, tapes, magnetic storage devices, and optical storage devices, MEMS, nano-technological storage device, etc.).

It is to be appreciated that the Detailed Description section, and not the Summary and Abstract sections, is intended to be used to interpret the claims. The Summary and Abstract sections may set forth one or more but not all exemplary embodiments of the present disclosure as contemplated by the inventor(s), and thus, are not intended to limit the present invention(s) and the appended claims in any way.

The present invention(s) have been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific embodiments will so fully reveal the general nature of the invention(s) that others can, by applying knowledge within the skill of the art, readily modify and/or adapt for various applications such specific embodiments, without undue experimentation, without departing from the general concept of the present invention(s). Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

The breadth and scope of the concepts of the present disclosure should not be limited by any of the above-described exemplary embodiments, but should be defined only in accordance with the following claims and their equivalents.

In the following, further examples are described to facilitate the understanding of the invention:
1. A method of tracking objects within video footage, the method comprising:
   receiving, at a computing device, video footage of a space and event data from a monitor, wherein the monitor comprises a motion sensor configured to measure movements of a monitored video object within the space, and the event data comprises motion events transmitted by the monitor;
   using a machine learning model on the computing device to identify, from the video footage, video events performed by the monitored video object; and
   assigning a respective persistent identifier to the monitored video object within the video footage based at least in part on commonality between video events performed by the monitored video object and motion events transmitted by the monitor.
2. The method of example 1, wherein the monitored video object is one among multiple video objects, the monitor is one among multiple monitors that each comprise a motion sensor configured to measure movements of a respective monitored video object, the method comprises assigning a respective persistent identifier to each of the multiple monitored video objects, and assigning the respective persistent identifiers comprises:
   assigning respective preliminary identifiers to the monitored video objects within the video footage at multiple different times within the video footage;
   finding preliminary identifiers assigned to a same monitored video object within different portions of the video footage based at least in part on commonality between video events performed by the same monitored video object and motion events transmitted by one of the monitors;
   consolidating preliminary identifiers found to be assigned to the same monitored video object into a single preliminary identifier; and
   after the consolidating step, converting the preliminary identifiers to the persistent identifiers.
3. The method of example 2, wherein assigning the respective preliminary identifiers comprises assigning a new preliminary identifier to a particular one of the monitored video objects at a discontinuity point within the video footage at which the computing device cannot determine, based on the video footage, identity between the particular one of the monitored video objects and any of the monitored video objects to which a preliminary identifier was assigned within an earlier portion of the video footage.
4. The method of example 3, wherein assigning the respective preliminary identifiers comprises:
   identifying key moments within the video footage, wherein every monitored video object is identifiable by the machine learning model from the video footage alone at each key moment;
   calculating similarities between motion events among the event data transmitted by each wearable monitor and video events performed by each monitored video object to which a preliminary identifier is assigned during at least one key moment.
5. The method of example 1, comprising processing motion data captured by the motion sensor of the wearable monitor to identify the motion events before transmitting the event data to the computing device.
6. The method of example 5, wherein the machine learning model is a first machine learning model, and the processing of the motion data comprises applying a second machine learning model hosted by the wearable monitor to the motion data.
7. The method of example 2, comprising identifying, for each persistent identifier, a corresponding one of the monitors that comprises the motion sensor configured to measure the movements of the monitored video object to which the persistent identifier is assigned.
8. The method of example 7, comprising applying a timestamp to one of the motion events transmitted by the corresponding one of the monitors matching a time when a corresponding video event is performed by the monitored video object to which the persistent identifier is assigned.
9. The method of example 1, comprising applying a timestamp to at least one of the motion events to match a time at which one of the video events occurred.
10. The method of example 9, wherein the event data comprises preliminary timestamps applied to the motion events by the monitor, and applying the timestamp to at least one of the motion events comprises replacing one of the preliminary timestamps with a final timestamp.
11. The method of example 2, wherein the video objects are athletes engaged in a sporting event.
12. The method of example 11, wherein each athlete wears one of the monitors.
13. The method of example 11, wherein the motion events comprise any one or any combination of kicking, running, walking, and standing.
14. The method of example 13, wherein the motion events comprise magnitude information in the form of any one or any combination of kick force, distance traveled, and speed.
15. The method of example 1, wherein the monitor comprises a controller configured to identify the motion events from the motion sensor of the monitor.
16. A system comprising:
   a wearable monitor comprising a motion sensor; and
   a computing device configured to receive event data transmitted by the wearable monitor, the event data comprising motion events, wherein the computing device comprises a non-transitory computer readable medium on which a machine learning model is stored, the machine learning model being configured to identify video events from video footage, and the computing device being configured to correlate the video events to the motion events.
17. The system of example 16, wherein the wearable monitor is configured to be integrated into an article of wear.
18. The system of example 17, wherein the article of wear is a shoe insole.
19. The system of example 16, wherein the wearable monitor comprises a motion sensor and a controller configured to identify the motion events from measurements acquired by the motion sensor.
20. The system of example 16, wherein the machine learning model is configured to track video objects within video footage based on the event data.

## Claims

1. A method of tracking objects within video footage, the method comprising:
receiving, at a computing device, video footage of a space and event data from a monitor, wherein the monitor comprises a motion sensor configured to measure movements of a monitored video object within the space, and the event data comprises motion events transmitted by the monitor;
using a machine learning model on the computing device to identify, from the video footage, video events performed by the monitored video object; and
assigning a respective persistent identifier to the monitored video object within the video footage based at least in part on commonality between video events performed by the monitored video object and motion events transmitted by the monitor.

2. The method of claim 1, wherein the monitored video object is one among multiple video objects, the monitor is one among multiple monitors that each comprise a motion sensor configured to measure movements of a respective monitored video object, the method comprises assigning a respective persistent identifier to each of the multiple monitored video objects, and assigning the respective persistent identifiers comprises:
assigning respective preliminary identifiers to the monitored video objects within the video footage at multiple different times within the video footage;
finding preliminary identifiers assigned to a same monitored video object within different portions of the video footage based at least in part on commonality between video events performed by the same monitored video object and motion events transmitted by one of the monitors;
consolidating preliminary identifiers found to be assigned to the same monitored video object into a single preliminary identifier; and
after the consolidating step, converting the preliminary identifiers to the persistent identifiers.

3. The method of claim 2, wherein assigning the respective preliminary identifiers comprises assigning a new preliminary identifier to a particular one of the monitored video objects at a discontinuity point within the video footage at which the computing device cannot determine, based on the video footage, identity between the particular one of the monitored video objects and any of the monitored video objects to which a preliminary identifier was assigned within an earlier portion of the video footage, wherein assigning the respective preliminary identifiers comprises preferably:
identifying key moments within the video footage, wherein every monitored video object is identifiable by the machine learning model from the video footage alone at each key moment;
calculating similarities between motion events among the event data transmitted by each wearable monitor and video events performed by each monitored video object to which a preliminary identifier is assigned during at least one key moment.

4. The method of one of the preceding claims, comprising processing motion data captured by the motion sensor of the wearable monitor to identify the motion events before transmitting the event data to the computing device, wherein the machine learning model is preferably a first machine learning model, and the processing of the motion data comprises applying a second machine learning model hosted by the wearable monitor to the motion data.

5. The method of claim 2 or 3, comprising identifying, for each persistent identifier, a corresponding one of the monitors that comprises the motion sensor configured to measure the movements of the monitored video object to which the persistent identifier is assigned, comprising preferably applying a timestamp to one of the motion events transmitted by the corresponding one of the monitors matching a time when a corresponding video event is performed by the monitored video object to which the persistent identifier is assigned.

6. The method of one of the preceding claims , comprising applying a timestamp to at least one of the motion events to match a time at which one of the video events occurred, wherein the event data comprises preferably preliminary timestamps applied to the motion events by the monitor, and applying the timestamp to at least one of the motion events comprises replacing one of the preliminary timestamps with a final timestamp.

7. The method of one of claims 2, 3 or 5, wherein the video objects are athletes engaged in a sporting event.

8. The method of claim 7, wherein each athlete wears one of the monitors.

9. The method of claim 7 or 8, wherein the motion events comprise any one or any combination of kicking, running, walking, and standing, wherein the motion events comprise preferably magnitude information in the form of any one or any combination of kick force, distance traveled, and speed.

10. The method of one of the preceding claims, wherein the monitor comprises a controller configured to identify the motion events from the motion sensor of the monitor.

11. A system comprising:
a wearable monitor comprising a motion sensor; and
a computing device configured to receive event data transmitted by the wearable monitor, the event data comprising motion events, wherein the computing device comprises a non-transitory computer readable medium on which a machine learning model is stored, the machine learning model being configured to identify video events from video footage, and the computing device being configured to correlate the video events to the motion events.

12. The system of claim 11, wherein the wearable monitor is configured to be integrated into an article of wear.

13. The system of claim 12, wherein the article of wear is a shoe insole.

14. The system of one of claims 11 to 13, wherein the wearable monitor comprises a motion sensor and a controller configured to identify the motion events from measurements acquired by the motion sensor.

15. The system of one of claims 11 to 14, wherein the machine learning model is configured to track video objects within video footage based on the event data.
